# EUROPEAN PATENT APPLICATION

(11) **EP 3 034 023 A1**
(43) Date of publication of application: **22.06.2016**
(21) Application number: 14836177.7
(22) Date of filing: 08.08.2014
(51) Int. Cl.: A61B 18/04

(54) **TREATMENT TOOL AND TREATMENT SYSTEM**

(30) Priority: 16.08.2013 US 201361866753 P
(71) Applicant: OLYMPUS CORPORATION, Shibuya-ku Tokyo 151-0072 (JP)
(72) Inventor: ICHIKAWA, Hiroaki, Shibuya-ku Tokyo 151-0072 (JP); KASAHARA, Hideyuki, Shibuya-ku Tokyo 151-0072 (JP); OISHI, Yusuke, Shibuya-ku Tokyo 151-0072 (JP)
(74) Representative: Schicker, Silvia
(86) International application number: PCT/JP2014/071072
(87) International publication number: WO 2015/022919

(57) **Abstract**

A treatment device applying energy to a biological tissue and treats it, includes: a first energy output portion disposed in one of a pair of holding portions and including a first operation region that removes the biological tissue as a treatment object, and a second operation region that is disposed around the first operation region and joins the biological tissues as the treatment objects when energy is applied to the first and second operation regions simultaneously; and a second energy output portion configured to apply energy to the biological tissue, disposed in the other holding portion of the holding portions, and disposed away from the second operation region more than from the first operation region when the holding portions are closed to hold the biological tissue between the holding portions, wherein a pressing force to be applied to the biological tissue between the second energy output portion and the first operation region is higher than a pressing force between the second energy output portion and the second operation region.

## Description

### Technical Field

This invention relates to a treatment device to treat a biological tissue by use of energy, and a treatment system.

### Background Art

For example, in Jpn. Pat. Appln. KOKAI Publication No. 2001-170069, there is disclosed a treatment device to treat a biological tissue by use of energy.

For example, in US 2010/042101 A1, there is disclosed a treatment device that is capable of joining biological tissues by supplying a conductive fluid such as saline to a specific region of the biological tissue and causing protein denaturation in the region.

For example, in US 2008/045944 A1, there is disclosed a treatment device which has a projecting portion including an insulating portion in the middle of a pair of electrodes and which is capable of preventing short circuit between the electrodes.

In a case where the biological tissues are joined to each other, a pressing force to bring joining surfaces of the biological tissues into contact closely with each other is required, and a temperature of about 110°C or more is required for the joining surfaces. When the temperature is higher than about 110°C, there is a tendency that a joining force increases, but it is known that decomposition (carbonization) of the biological tissues is rapidly advanced until the biological tissues reach about 280°C. Consequently, when the biological tissues are heated up to 280°C, the joining and incision of the biological tissues can simultaneously be performed, and the need for mechanical incision of the joining surfaces is eliminated.

In the treatment device disclosed in Jpn. Pat. Appln. KOKAI Publication No. 2001-170069, US 2010/042101 A1 or US 2008/045944 A1, if a temperature of a holding surface of the biological tissue can be raised up to 280°C, there is a high possibility that a decomposed region (an incised region) of the biological tissue is disadvantageously larger than a joining area between the biological tissues. Therefore, in the abovementioned treatment devices, the joining and the incision of the biological tissues can simultaneously be performed, but it is difficult to obtain a high joining force between the biological tissues.

### Summary of Invention

An object of this invention is to provide a treatment device and a treatment system that is capable of simultaneously performing joining and incision of biological tissues as treatment objects and additionally capable of obtaining a high joining force between the biological tissues as the treatment objects in a case where the biological tissues are treated by using energy.

According to one embodiment of the present invention, a treatment device which applies energy to a biological tissue to treat the biological tissue, includes: a pair of holding portions that are relatively openable and closable and configured to hold the biological tissue; a first energy output portion that is disposed in one of the holding portions to face the other holding portion and includes a first operation region that operates to remove the biological tissue as a treatment object when energy is applied, and a second operation region that is disposed around the first operation region and operates to join the biological tissues as the treatment objects when the energy is applied to the second operation region simultaneously with the first operation region; and a second energy output portion that is disposed in the other holding portion of the holding portions to face the first energy output portion, and that is disposed away from the second operation region more than from the first operation region when the pair of holding portions are relatively closed to hold the biological tissue between the holding portions, wherein a pressing force to be applied to the biological tissue between the second energy output portion and the first operation region is higher than a pressing force to be applied to the biological tissue between the second energy output portion and the second operation region, and the second energy output portion is configured to apply the energy to the biological tissue.

### Brief Description of Drawings

FIG. 1 is a schematic view showing a treatment system according to first to eighth embodiments;
FIG. 2 is a schematic block diagram of the treatment system according to the first to eighth embodiments;
FIG. 3A is a schematic plan view showing a first treatment section of a treatment device of the treatment system according to the first embodiment;
FIG. 3B is a schematic transverse cross-sectional view showing a state where a biological tissue as a treatment object is held by a treatment section of the treatment device of the treatment system according to the first embodiment taken along the 3B-3B line in FIG. 3A;
FIG. 3C is a schematic transverse cross-sectional view showing a state where the biological tissue as the treatment object is held between a first energy output portion and a second energy output portion of the treatment section of the treatment device of the treatment system according to the first embodiment;
FIG. 4 is a schematic view showing a modification of the treatment device of the treatment system according to the first to eighth embodiments;
FIG. 5A is a schematic transverse cross-sectional view showing a state where the biological tissue as the treatment object is held by a treatment section of the treatment device of the treatment system according to the second embodiment;
FIG. 5B is a schematic transverse cross-sectional view showing a state where the biological tissue as the treatment object is held between a first energy output portion and a second energy output portion of the treatment section of the treatment device of the treatment system according to the second embodiment;
FIG. 6 is a schematic transverse cross-sectional view showing a state where the biological tissue as the treatment object is held between a first energy output portion and a second energy output portion of a treatment section of the treatment device of the treatment system according to the third embodiment;
FIG. 7 is a schematic transverse cross-sectional view showing a state where the biological tissue as the treatment object is held between a first energy output portion and a second energy output portion of a treatment section of the treatment device of the treatment system according to the fourth embodiment;
FIG. 8 is a schematic transverse cross-sectional view showing a state where the biological tissue as the treatment object is held between a first energy output portion and a second energy output portion of a treatment section of the treatment device of the treatment system according to the fifth embodiment;
FIG. 9A is a schematic perspective view showing first and second energy output portions of a treatment section of the treatment device of the treatment system according to the sixth embodiment;
FIG. 9B is a schematic side view of the treatment section of the treatment device of the treatment system according to the sixth embodiment;
FIG. 9C is a schematic transverse cross-sectional view of the treatment section of the treatment device of the treatment system according to the sixth embodiment taken along the 9C-9C line in FIG. 9B;
FIG. 9D is a schematic transverse cross-sectional view of the treatment section of the treatment device of the treatment system according to the sixth embodiment taken along the 9D-9D line in FIG. 9B;
FIG. 9E is a schematic cross-sectional view of the treatment section of the treatment device of the treatment system according to the sixth embodiment taken along the 9E-9E line in FIG. 9B;
FIG. 10 is a schematic perspective view showing an energy output portion of one of a pair of treatment sections of the treatment device of the treatment system according to the seventh embodiment;
FIG. 11 is a schematic perspective view showing one of a pair of treatment sections of the treatment device of the treatment system according to the eighth embodiment;
FIG. 12 is a schematic view showing a treatment system according to a ninth embodiment; and
FIG. 13 is a schematic transverse cross-sectional view showing a state where the biological tissue as the treatment object is held between a first energy output portion and a second energy output portion of a treatment section of a treatment device of the treatment system according to the ninth embodiment.

### Description of Embodiments

Hereinafter, embodiments of this invention will be described with reference to the drawings.

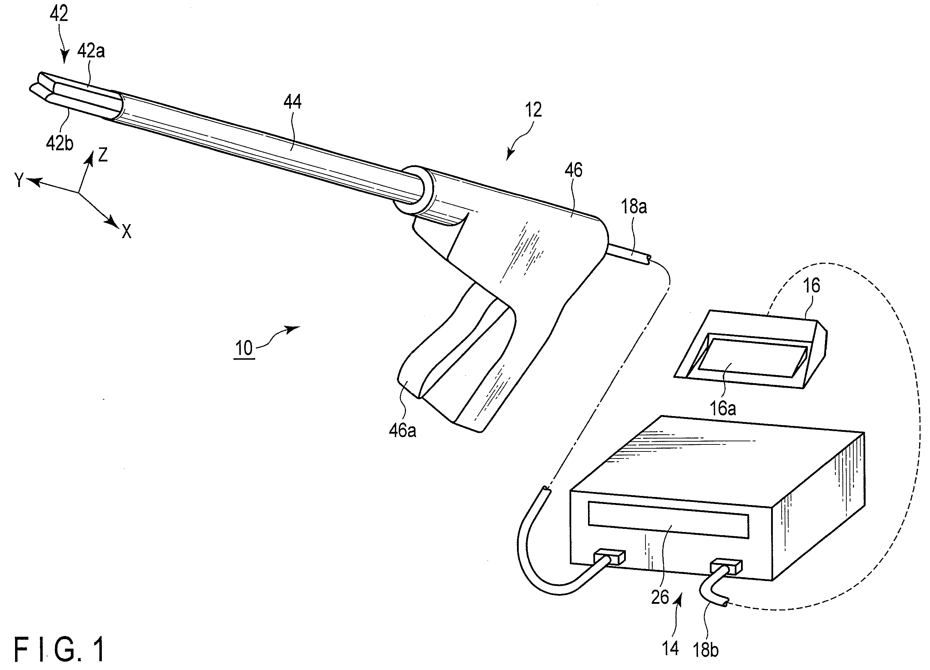

A first embodiment will be described with reference to FIG. 1 to FIG. 3C.

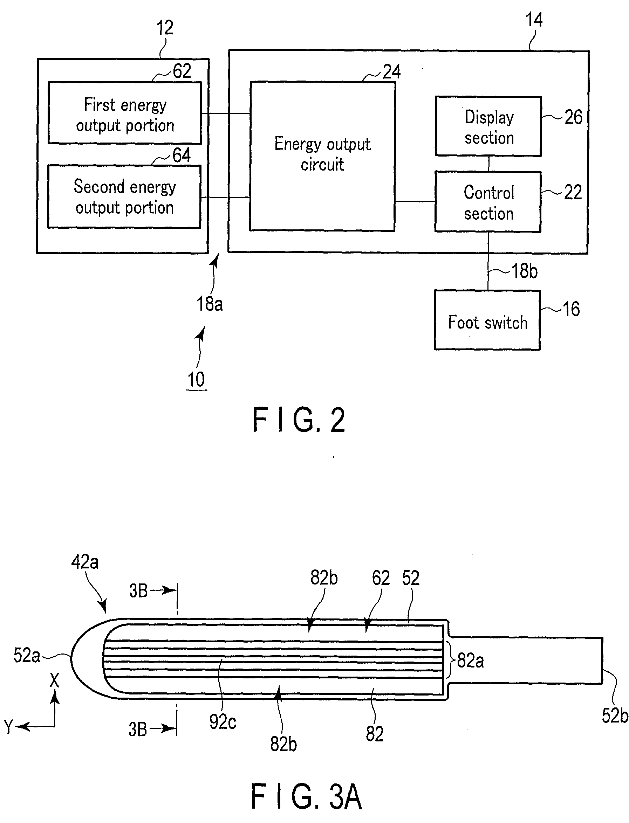

As shown in FIG. 1, a treatment system (an energy treatment apparatus) 10 according to this embodiment includes a treatment device (an energy treatment device) 12, and a controller 14 that applies energy to the treatment device 12. The controller 14 controls temperatures of after-mentioned first and second energy output portions 62 and 64 (see FIG. 2) of the treatment device 12 at suitable temperatures.

The controller 14 is connected to a foot switch 16 having a pedal 16a that switches ON/OFF states of heat energy to be applied to the treatment device 12. The treatment device 12 is electrically connected to the controller 14 by a first cable 18a in which lead wires or signal lines are bundled. The controller 14 is connected to the foot switch 16 by a second cable 18b in which lead wires or signal lines are bundled. The foot switch 16 is capable of inputting a signal into the controller 14 by an operation of the pedal 16a, or the like, and the controller 14 is capable of controlling the energy to be applied to the treatment device 12 on the basis of the operation of the pedal 16a of the foot switch 16, or the like.

As shown in FIG. 2, the controller 14 includes a control section (CPU) 22, an energy output circuit (a heating portion drive circuit) 24, and a display section 26. The energy output circuit 24 and the display section 26 are controlled by the control section 22. The display section 26 is for use in displaying a state of the controller 14 or in performing various settings. As the display section 26, for example, a touch panel is preferably used.

It is to be noted that, when the foot switch 16 is connected to the control section 22 and the pedal 16a of the foot switch 16 is pressed, the energy output circuit 24 simultaneously outputs the energy to the first and second energy output portions 62 and 64 which will be described later.

As shown in FIG. 1, the treatment device 12 includes a treatment section 42 that treats a biological tissue L, an insertion section 44, and an operation section 46.

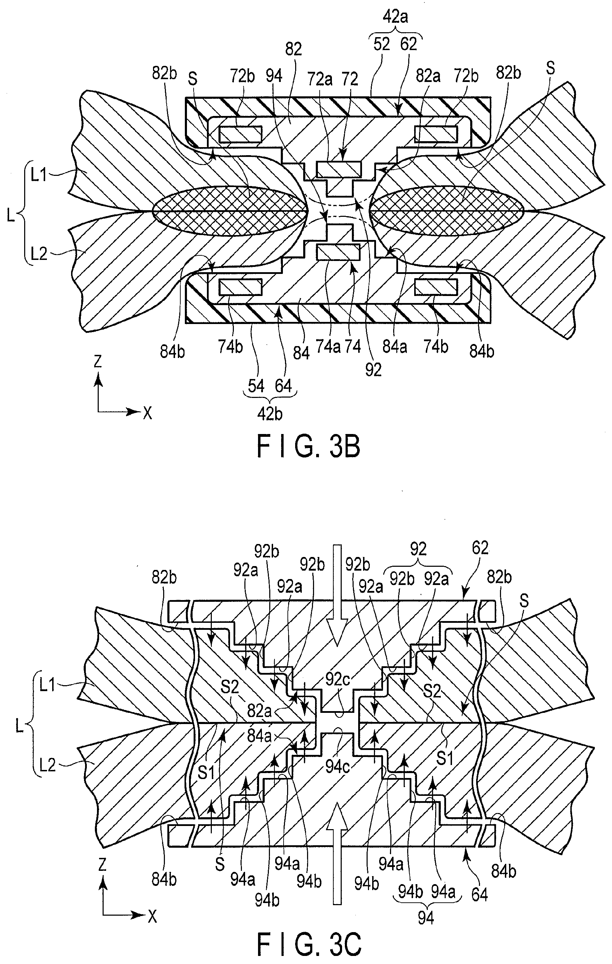

As shown in FIG. 3B, the treatment section 42 includes a pair of jaws (first and second jaws) 52 and 54 that are openable and closable as holding portions of the biological tissue, and the energy output portions (the first and second energy output portions) 62 and 64 disposed in the jaws 52 and 54. The first jaw (a first holding portion) 52 and the first energy output portion 62 form a first treatment section 42a (see FIG. 1) shown in FIG. 3A, and the second jaw (a second holding portion) 54 and the second energy output portion 64 form a second treatment section 42b (see FIG. 1). It is to be noted that in this embodiment, the first and second treatment sections 42a and 42b are formed symmetrically to a plane formed by a longitudinal direction Y and a width direction X which will be described later (see FIG. 1 and FIG. 3A to FIG. 3C).

The first energy output portion 62 includes a first heating portion 72 such as a resistance heating heater, and a first heat transfer plate 82 that transfers heat generated by the first heating portion 72 to the biological tissue. The second energy output portion 64 includes a second heating portion 74 such as a resistance heating heater, and a second heat transfer plate 84 that transfers heat generated by the second heating portion 74 to the biological tissue.

As shown in FIG. 3A, the first jaw 52 has a longitudinal axis (the longitudinal direction) Y defined by a distal portion 52a and a proximal portion 52b, and the width direction X defined in a direction perpendicular to the longitudinal axis Y. The first jaw 52 is formed into a substantially flat plate shape that is long along the longitudinal direction Y of the insertion section 44 and that is smaller in the width direction X perpendicular to the longitudinal direction Y than in the longitudinal direction Y. The second jaw 54 is formed symmetrically or substantially symmetrically to the first jaw 52. The first and second jaws 52 and 54 are opened and closed by an operation of an opening/closing lever 46a of the operation section 46 in a known mechanism. That is, when the opening/closing lever 46a is operated, the first and second jaws 52 and 54 are opened and closed in a Z-direction shown in FIG. 3B and FIG. 3C by known means such as a wire or a rod disposed in the insertion section 44. It is to be noted that one of the first and second jaws 52 and 54 may be moved or both of them may be moved by the operation of the opening/closing lever 46a of the operation section 46 in the known mechanism. That is, the first and second jaws 52 and 54 are relatively openable and closable.

As the first and second jaws 52 and 54, for example, a ceramic material, a resin having a heat resistance and electric insulating properties, a metal material treated in an insulating manner, or the like is preferably used. Additionally, the first and second jaws 52 and 54 are preferably made of a material having heat insulating properties.

The energy output circuit 24 is able to control surface temperatures of the first and second energy output portions 62 and 64 of the treatment device 12. Specifically, when the energy output circuit 24 applies the energy to the first and second heating portions 72 and 74 to heat the first and second heating portions 72 and 74 and the heat (the heat energy) is transferred to the first and second heat transfer plates 82 and 84, temperatures of surfaces (biological tissue holding surfaces) of the first and second heat transfer plates 82 and 84 can be controlled. Further, the heat (the heat energy) is transferred to biological tissues L1 and L2 as treatment objects through the surfaces of the first and second heat transfer plates 82 and 84, to dehydrate the biological tissues L1 and L2. That is, the treatment device 12 according to this embodiment is controlled by the controller 14 and is therefore able to output the heat energy from the first energy output portion 62 and the second energy output portion 64. Further, the heat energy is exerted to the biological tissue L from the first energy output portion 62 and the second energy output portion 64 to treat the biological tissue L.

As the first and second heating portions 72 and 74, heating elements (micro heaters) may be used, or plate-like heaters may be used. When the first and second heating portions 72 and 74 are the heating elements, the heating portions are preferably disposed or buried in back surfaces of the first and second heat transfer plates 82 and 84, and when the heating portions are the plate-like heaters, the heating portions are preferably disposed on the back surfaces of the first and second heat transfer plates 82 and 84. The first and second heating portions 72 and 74 suitably have a bar shape that is long in the longitudinal direction Y of the first and second heat transfer plates 82 and 84 or the direction (the width direction X) perpendicular to the longitudinal direction Y.

As the first and second heat transfer plates 82 and 84, a metal material such as a stainless steel alloy or a suitably conductive material such as a fine ceramic material of silicon nitride is used. As shown in FIG. 3B and FIG. 3C, the first and second heat transfer plates 82 and 84 face each other, and are used as holding surfaces (grasping surfaces) of the biological tissue L of the treatment object.

It is to be noted that in FIG. 3C, the first jaw 52 and the first and second heating portions 72 and 74 in FIG. 3B are omitted. On the other hand, FIG. 3B shows after-mentioned first and third operation regions 82a and 84a in detail.

As shown in FIG. 3A to FIG. 3C, the first heat transfer plate 82 of the first energy output portion 62 includes the first operation region (a holding surface) 82a of a middle in its width direction (an X-direction), and a pair of second operation regions (holding surfaces) 82b disposed around the first operation region 82a (end portions in the width direction X). That is, the first operation region 82a is sandwiched between the pair of second operation regions 82b along the width direction X. The first and second operation regions 82a and 82b are continuous along the width direction X. The first operation region 82a projects toward the second heat transfer plate 84, i.e., the second energy output portion 64 more than the pair of second operation regions 82b.

The second heat transfer plate 84 includes the third operation region (holding surface) 84a of a middle in the width direction, and fourth operation regions (holding surfaces) 84b disposed around the third operation region 84a. That is, the third operation region 84a is sandwiched between a pair of fourth operation regions 84b along the width direction X. The third and fourth operation regions 84a and 84b are continuous along the width direction X. The third operation region 84a projects toward the first heat transfer plate 82, i.e., the first energy output portion 62 more than the pair of fourth operation regions 84b.

As shown in FIG. 3B, in this embodiment, the first heating portion 72 includes a first heating element 72a disposed in the first operation region 82a of the middle in the width direction, and a pair of second heating elements 72b disposed in the second operation regions 82b around the first operation region 82a. Sets of the first heating element 72a and the pair of second heating elements 72b are preferably disposed along the longitudinal axis Y. Here, as to the first heating element 72a, in a state where the pedal 16a of the foot switch 16 is pressed, the controller 14 raises a temperature of a surface (a holding surface) of a top portion 92c of the first operation region 82a of the first heat transfer plate 82 to about 280°C in, e.g., time t1 (e.g., optional time of several ten seconds to several minutes or the like), and the temperature is controlled to be maintained. As to the pair of second heating elements 72b, in the state where the pedal 16a of the foot switch 16 is pressed, the controller 14 raises a temperature of a surface of the second operation region 82b of the first heat transfer plate 82 to about 200°C in, e.g., time t2 (e.g., optional time of several ten seconds to several minutes or the like), and the temperature is controlled to be maintained. It is to be noted that the first heating element 72a and the pair of second heating elements 72b simultaneously start to raise the temperatures.

The second heating portion 74 includes a third heating element 74a disposed in the third operation region 84a of the middle in the width direction, and a pair of fourth heating elements 74b disposed in the fourth operation regions 84b in a periphery of the third operation region 84a. Here, as to the third heating element 74a, in the state where the pedal 16a of the foot switch 16 is pressed, the controller 14 raises a temperature of a surface (a holding surface) of a top portion 94c of the third operation region 84a of the second heat transfer plate 84 at about 280°C in, e.g., the time t1 (e.g., the optional time of several ten seconds to several minutes or the like), and the temperature is controlled to be maintained. As to the pair of fourth heating elements 74b, in the state where the pedal 16a of the foot switch 16 is pressed, the controller 14 raises a temperature of a surface of the fourth operation region 84b of the second heat transfer plate 84 at about 200°C in, e.g., the time t2 (e.g., the optional time of several ten seconds to several minutes or the like), and the temperature is controlled to be maintained. It is to be noted that the third heating element 74a and the pair of fourth heating elements 74b simultaneously start to raise the temperatures.

It is to be noted that the first to fourth heating elements 72a, 72b, 74a and 74b preferably simultaneously start to raise the temperatures.

As shown in FIG. 3B and FIG. 3C, in this embodiment, steps are formed in the first and third operation regions 82a and 84a. The first operation region 82a includes steps 92 in the width direction X. As shown in FIG. 3C, the steps 92 of the first operation region 82a have first facing surfaces 92a disposed toward the second heat transfer plate 84 facing the first facing surfaces 92a, and first erected surfaces 92b perpendicular to the first facing surfaces 92a and disposed toward the second operation regions 82b, i.e., an outer side of the treatment section 42 in the width direction X. The first facing surfaces 92a are adjacent to the first erected surfaces 92b, and the first facing surfaces 92a come close to the second heat transfer plate 84 as the first erected surfaces 92b are substantially disposed toward the middle in the width direction X. Especially, the first operation region 82a of the substantially middle of the width direction X of the first treatment section 42a includes the top portion 92c closest to the second heat transfer plate 84 substantially in the middle of the width direction X (the middle of the first operation region 82a). That is, the first operation region 82a projects toward the second energy output portion 64 more than the second operation regions 82b. It is to be noted that the first facing surfaces 92a, the first erected surfaces 92b and the top portion 92c form a biological tissue holding surface of the first operation region 82a.

Here, for the convenience of description, it is described that the first facing surfaces 92a are perpendicular to the first erected surfaces 92b, but the first facing surfaces 92a and the first erected surfaces 92b do not necessarily have to be perpendicular to one another as long as the surfaces are shaped in the form of the steps.

The third operation region 84a includes steps 94 in the width direction X. The steps 94 of the third operation region 84a have second facing surfaces 94a disposed toward the first heat transfer plate 82, and second erected surfaces 94b perpendicular to the second facing surfaces 94a and disposed toward the fourth operation regions 84b, i.e., the outer side of the treatment section 42 in the width direction X. The second facing surfaces 94a are adjacent to the second erected surfaces 94b, and the second facing surfaces 94a come close to the first heat transfer plate 82 as the second erected surfaces 94b are substantially disposed toward the middle in the width direction X. Especially, the third operation region 84a of the substantially middle of the width direction X of the second treatment section 42b includes the top portion 94c closest to the first heat transfer plate 82 substantially in the middle of the width direction X (substantially in the middle of the third operation region 84a). That is, the third operation region 84a projects toward the first energy output portion 62 more than the fourth operation regions 84b. It is to be noted that the second facing surfaces 94a, the second erected surfaces 94b and the top portion 94c form a biological tissue holding surface of the third operation region 84a.

Here, for the convenience of description, it is described that the second facing surfaces 94a are perpendicular to the second erected surfaces 94b, but the second facing surfaces 94a and the second erected surfaces 94b do not necessarily have to be perpendicular to one another as long as the surfaces are shaped in the form of the steps.

A length (a width) of the width direction X of the first and second facing surfaces 92a and 94a is preferably, for example, about 0.3 mm, and heights of the first and second erected surfaces 92b and 94b are preferably, for example, about 0.3 mm, but these values can suitably be set. That is, the heights of the first and second erected surfaces 92b and 94b may be larger than the widths of the first and second facing surfaces 92a and 94a, and the heights of the first and second erected surfaces 92b and 94b may be smaller than the widths of the first and second facing surfaces 92a and 94a.

The second operation regions 82b function as the biological tissue holding surfaces disposed toward the second heat transfer plate 84. That is, the pair of second operation regions 82b function as a pair of holding surfaces holding the biological tissues, respectively. The fourth operation regions 84b function as the biological tissue holding surfaces disposed toward the first heat transfer plate 82. That is, the pair of fourth operation regions 84b function as a pair of holding surfaces holding the biological tissues, respectively. The second and fourth operation regions 82b and 84b preferably face one another in parallel.

In a case where the widths of the first and second operation regions 82a and 82b in the width direction X are compared with each other, the combined width of the pair of second operation regions 82b in the width direction X is preferably larger than the width of the first operation region 82a in the width direction X.

Next, an operation of the treatment system 10 according to this embodiment will be described.

The treatment section 42 is disposed opposite to the biological tissues L (L1 and L2) as joining and cutting objects while holding the operation section 46 with one hand. When the biological tissues L (L1 and L2) of the joining and cutting objects are present in a tubular hole, the treatment section 42 and the insertion section 44 are inserted into the tubular hole to dispose the treatment section 42 opposite to the biological tissues L (L1 and L2) as the joining and cutting objects.

The opening/closing lever 46a is operated to open the pair of jaws 52 and 54. Further, the jaws 52 and 54 are closed to hold the biological tissues L1 and L2 as the treatment objects between the first heat transfer plate 82 and the second heat transfer plate 84 as shown in FIG. 3A and FIG. 3B.

At this time, a distance between the first operation region 82a of the first heat transfer plate 82 of the first energy output portion 62 and the third operation region 84a of the second heat transfer plate 84 of the second energy output portion 64 is smaller than a distance between the second operation region 82b of the first heat transfer plate 82 of the first energy output portion 62 and the fourth operation region 84b of the second heat transfer plate 84 of the second energy output portion 64. Further, the first and second facing surfaces 92a and 94a of the first and third operation regions 82a and 84a are present in the same direction as an opening/closing direction of the jaws 52 and 54. Consequently, a larger pressing force is applied to a boundary between the biological tissues L1 and L2 by each portion between the first facing surface 92a and the second facing surface 94a as the surfaces of the first and second heat transfer plates 82 and 84. The first heat transfer plate 82 is disposed close to the third operation region 84a of the second heat transfer plate 84 and disposed away from the fourth operation regions 84b more than from the third operation region 84a. The second heat transfer plate 84 is disposed close to the first operation region 82a of the first heat transfer plate 82 and disposed away from the second operation regions 82b more than from the first operation region 82a. Consequently, the largest pressing force is applied to the boundary between the biological tissues L1 and L2, especially in a portion between the top portion 92c of the first operation region 82a and the top portion 94c of the third operation region 84a and in the vicinity of the portion. In consequence, a holding force to hold the biological tissues L1 and L2 can be increased toward a middle side of the treatment section 42 in the width direction X as compared with the outer side in the width direction. Therefore, the biological tissues L1 and L2 come in contact closely with each other in the boundary therebetween toward the middle side of the treatment section 42 in the width direction X as compared with the outer side in the width direction X.

The second and fourth operation regions 82b and 84b are present in the same direction as the opening/closing direction of the jaws 52 and 54. Consequently, the larger pressing force is applied to the boundary of the biological tissues L1 and L2 between the facing surfaces 92a and 94a as the surfaces of the first and second heat transfer plates 82 and 84. Further, the largest pressing force is applied to the boundary between the biological tissues L1 and L2 especially in the top portion 92c of the first operation region 82a, the top portion 94c of the third operation region 84a and the vicinities of the top portions.

Here, in a state where the biological tissue is held between the first energy output portion 62 and the second energy output portion 64, the controller 14 controls the first operation region 82a and the second energy output portion 64 at a temperature at which the biological tissue between the first operation region 82a and the second energy output portion 64 is removed in the biological tissues. Additionally, in the state where the biological tissue is held between the first energy output portion 62 and the second energy output portion 64, the controller 14 controls the second operation regions 82b and the second energy output portion 64 at a temperature at which the biological tissues between the second operation region 82b and the second energy output portion 64 are joined in the biological tissues.

The surface temperatures of the top portions 92c and 94c of the first operation region 82a of the first heat transfer plate 82 and the third operation region 84a of the second heat transfer plate 84 are controlled at about 280°C. Consequently, outer surfaces of the biological tissues L1 and L2 sandwiched between the top portions 92c and 94c are heated up to 280°C. The boundary of the biological tissues L1 and L2 sandwiched between the top portions 92c and 94c is heated up to about 280°C. Consequently, the boundary of the biological tissues L1 and L2 sandwiched between the top portions 92c and 94c is carbonized to be decomposed, i.e., burnt out to be incised by the heat transfer plates 82 and 84 whose surface temperatures are 280°C. In other words, the biological tissues L1 and L2 sandwiched between the top portions 92c and 94c are removed by inputting the energy into the biological tissue itself between the top portions 92c and 94c.

It is to be noted that the surface temperatures of the top portions 92c and 94c are controlled at about 280°C, and hence the biological tissue in the vicinity of the biological tissue sandwiched between the top portions 92c and 94c is carbonized to be decomposed, i.e., burnt out to be incised by the heat transferred from the transfer plates 82 and 84 whose surface temperatures are 280°C. In other words, the biological tissue in the vicinity of the biological tissue sandwiched between the top portions 92c and 94c is removed by inputting the energy into the biological tissue between the top portions 92c and 94c. A range to be removed is changed in accordance with a size of the energy to be input, a component of the biological tissue, or the like.

A distance between the first facing surface 92a and the second facing surface 94a which face each other increases as the surfaces are away from the biological tissue sandwiched between the top portions 92c and 94c along the width direction X. Consequently, a holding pressure of the biological tissue between the first facing surface 92a and the second facing surface 94a which face each other is gradually decreased as the biological tissue is away from the top portions 92c and 94c along the width direction X. Therefore, even between the first operation region 82a and the third operation region 84a, a close contact force between the biological tissues L1 and L2 is lower than that between the biological tissues L1 and L2 sandwiched between the top portions 92c and 94c as the biological tissue is away from the top portions 92c and 94c along the width direction X. In consequence, even temperatures of the biological tissues between the first operation region 82a and the third operation region 84a do not rise up to a temperature (e.g., 280°C) of the boundary of the biological tissues sandwiched between the top portions 92c and 94c as the biological tissue is away from the biological tissue sandwiched between the top portions 92c and 94c in the width direction X. Consequently, even between the first operation region 82a and the third operation region 84a, the temperature of the boundary of the biological tissues L1 and L2 rises to the temperature at which the biological tissues are joined, but does not reach a temperature at which the biological tissues are carbonized, i.e., decomposed as the biological tissue is away from the biological tissue sandwiched between the top portions 92c and 94c.

Additionally, a boundary portion between the first facing surface 92a and the first erected surface 92b and a boundary portion between the second facing surface 94a and the second erected surface 94b are hard to come in contact with the biological tissues as the portions are away from the top portions 92c and 94c along the width direction X. Consequently, there is generated a portion in which the heat is hard to be transferred from the heat transfer plates 82 and 84 directly even to the biological tissues between the first operation region 82a and the third operation region 84a. Therefore, even the biological tissues between the first operation region 82a and the third operation region 84a are prevented from being heated up to a temperature at which the biological tissues are decomposed as the biological tissues are away from the top portions 92c and 94c in the width direction X.

Thus, even between the first operation region 82a and the third operation region 84a, a boundary portion of the biological tissues L1 and L2 is prevented from reaching the decomposition temperature in excess of the joining temperature as the biological tissue is away from the top portions (middles in the width direction) 92c and 94c in the width direction. Therefore, at a position away from the top portions (the middles in the width direction) 92c and 94c in the width direction, the boundary of the biological tissues L1 and L2 between the first operation region 82a and the third operation region 84a can be adjusted into a joined state (a coagulated state).

In third and fourth facing surfaces 92 and 94 of the second and fourth operation regions 82b and 84b, the temperatures of the outer surfaces of the biological tissues are heated up to about 200°C at positions away from the top portions 92c and 94c in the width direction X. At this time, the third and fourth facing surfaces 92 and 94 of the second and fourth operation regions 82b and 84b are flat surfaces which face each other, and hence the surfaces come in contact closely with the outer surfaces of the biological tissues, and the joining surface between the biological tissues is heated up to about 200°C. In consequence, the second and fourth operation regions 82b and 84b can be heated up to the temperature suitable for the joining of the biological tissues L1 and L2 to each other.

Therefore, according to the treatment system 10 of this embodiment, in the part between the top portions 92c and 94c of the first and third operation regions 82a and 84a and the vicinity of the part, the biological tissue can be decomposed and heated in a state similar to the state where the biological tissue is cut. On the other hand, even between the first operation region 82a and the third operation region 84a, the boundary of the biological tissues L1 and L2 can be heated up to a temperature suitable for the joining in a state where the biological tissues are brought into contact closely with each other as the biological tissue is away from the biological tissue between the top portions 92c and 94c along the width direction X in the biological tissues between the second operation region 82b and the fourth operation region 84b. The first and third operation regions 82a and 84a are formed as stairs from the top portions 92c and 94c as tops, and hence the decomposition, i.e., the cutting as well as the joining of the biological tissues can be performed by one energy output. At this time, a joining region S of the biological tissue is not limited to the biological tissue between the second operation region 82b and the fourth operation region 84b, but can be enlarged to a part of the biological tissue between the first operation region 82a and the third operation region 84a which is continuous with the biological tissue between the second operation region 82b and the fourth operation region 84b. Therefore, the biological tissues can be joined to each other in a broad range along the width direction X. Needless to say, the biological tissues are joined to each other in a broad range not only in the width direction X but also in the axial direction Y. In consequence, according to the treatment system 10 of this embodiment, a strong joining force can be exerted to join the biological tissues to each other.

It is to be noted that in the treatment device 12 which applies the energy to the biological tissues to treat the biological tissues, especially the pressing force between the top portions 92c and 94c of the first and third operation regions 82a and 84a is higher than a pressing force at a position away from the top portions 92c and 94c and a pressing force between the second operation region 82b and the fourth operation region 84b. Consequently, the energy can efficiently be applied especially to a region to be cut between the top portions 92c and 94c of the first and third operation regions 82a and 84a in the biological tissues. Therefore, the treatment device 12 efficiently cuts the biological tissue as the treatment object, and a joining area of the biological tissues around a cutting section can be increased.

It is to be noted that FIG. 3A and FIG. 3B show that a part of each joining region S is positioned on an outer side from an end portion of each of the heat transfer plates 82 and 84 in the width direction X, but the joining region S is also suitably present on an inner side from the end portion of the treatment section 42 in the width direction X. In this case, a range of the biological tissue which is influenced by the heat can be defined. For example, when an edge portion of the first jaw 52 is disposed closer to an edge portion of the second jaw 54 than the second operation region 82b and the edge portion of the second jaw 54 is disposed closer to the edge portion of the first jaw 52 than the fourth operation region 84b, the pressing force in the vicinity of the outer side of each of the second and fourth operation regions 82b and 84b in the width direction X is decreased and the close contact force between the biological tissues can be decreased. In consequence, it is possible to prevent the close contact between the biological tissues on an outer side from an outer edge of each of the first and second jaws 52 and 54, and the range of each joining surface can be adjusted on an inner side from the outer edge of each of the first and second jaws 52 and 54.

It is to be noted that in this embodiment, there has been described an example where in the first energy output portion 62, the first heating portion 72, e.g., the resistance heating heater or the like (the first heating element 72a and the pair of second heating elements 72b) is used as shown in FIG. 3A, but there may be used one planar heater or planar heaters controllable at different temperatures along the width direction X.

Additionally, in this embodiment, there has been described an example where the heating portions 72 and 74, e.g., the heaters are used to heat the heat transfer plates 82 and 84, but the present invention is not limited to the heaters, and light, high frequency electrodes or the like may be used as long as the temperature to be applied to the biological tissue can be controlled. This also applies to after-mentioned embodiments. Additionally, when the biological tissue is treated, needless to say, heat energy, light energy and high frequency energy by the heating portions 72 and 74 may suitably be combined.

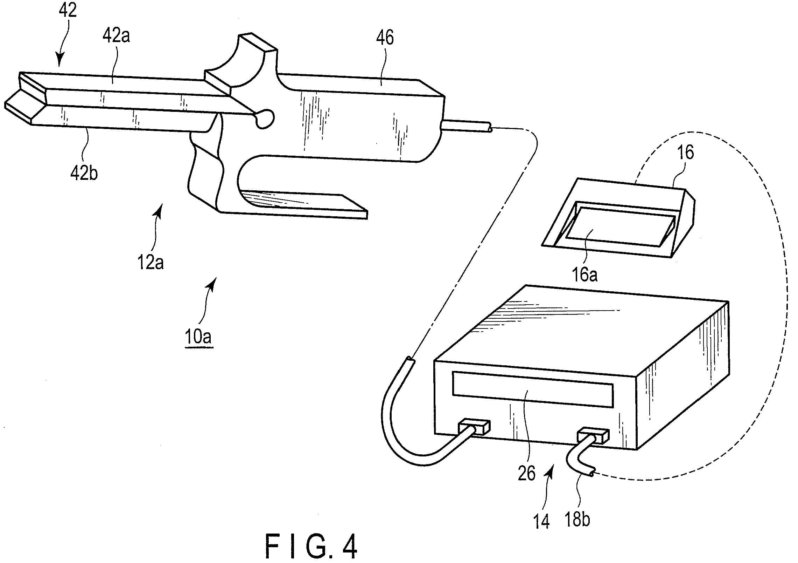

It is to be noted that in this embodiment, an example where the treatment device 12 includes the insertion section 44 has been described, but the insertion section 44 is not necessarily required as in a treatment device 12a shown in FIG. 4.

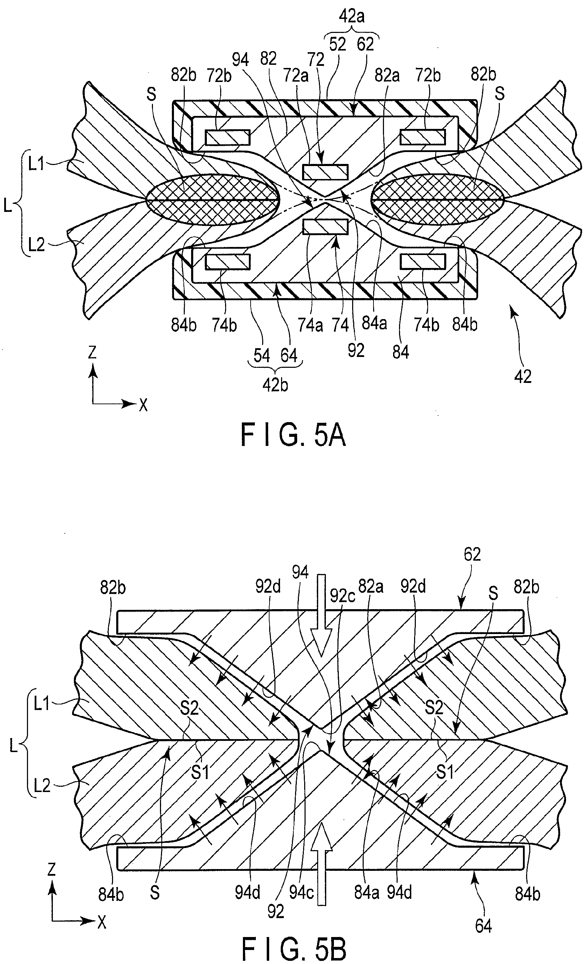

Next, a second embodiment will be described with reference to FIG. 5A and FIG. 5B. This embodiment is a modification of the first embodiment, the same members or members having the same functions as those described in the first embodiment are denoted with the same reference signs, and detailed description is omitted.

As shown in FIG. 5A and FIG. 5B, a first operation region 82a of a first energy output portion 62 according to this embodiment is not formed of steps, but is formed as gently inclined surfaces 92d inclined from a top portion 92c of a middle in a width direction (an X-direction) toward second operation regions 82b on outer sides in the width direction X. Similarly, a third operation region 84a of a second energy output portion 64 is not formed of steps, but is formed as gently inclined surfaces 94d inclined from a top portion 94c of the middle in the width direction (the X-direction) toward fourth operation regions 84b in the width direction X.

When biological tissues L1 and L2 are held between the first operation region 82a and the third operation region 84a, a pressing force is applied at a position that shifts from the top portions 92c and 94c in the width direction X, in a direction (a vertical direction to a tissue holding surface) different from that of a pressure to be applied in an upward-downward direction (a Z-direction). At this time, when the biological tissues are held between the first operation region 82a and the third operation region 84a excluding the top portions 92c and 94c, as described in the first embodiment, a smaller pressing force is applied than the pressing force to be applied in the upward-downward direction (the Z-direction).

A holding force of the biological tissues between the top portions 92c and 94c is larger than that in another region. Additionally, when the biological tissues are treated, temperatures of the top portions 92c and 94c and their vicinities are controlled to rise up to about 280°C. Therefore, the biological tissues L1 and L2 sandwiched between the top portions 92c and 94c are decomposed, i.e., burnt out to be incised by heat transfer plates 82 and 84 whose surface temperatures are 280°C. In consequence, the biological tissues L1 and L2 sandwiched between the top portions 92c and 94c are removed by inputting energy thereinto.

A distance between the inclined surfaces 92d and 94d increases as the surfaces are away from the biological tissues sandwiched between the top portions 92c and 94c along the width direction X. Additionally, as described above, the pressing force onto the biological tissues L1 and L2 is loaded in the vertical direction to the inclined surfaces 92d and 94d, and hence a sandwiching pressure of the biological tissues is gradually decreased as the biological tissue is away from the top portions 92c and 94c along the width direction X. Therefore, even between the first operation region 82a and the third operation region 84a, a close contact force between the biological tissues L1 and L2 also decreases at a position that shifts from the biological tissue between the top portions 92c and 94c along the width direction X. Therefore, even between the first operation region 82a and the third operation region 84a, a temperature is hard to rise up to a temperature (e.g., 280°C) of the biological tissue sandwiched between the top portions 92c and 94c as the biological tissue is away from the biological tissue sandwiched between the top portions 92c and 94c along the width direction X. Consequently, even between the first operation region 82a and the third operation region 84a, the biological tissues L1 and L2 are joined to each other, but are not decomposed, i.e., are not removed as the biological tissues are away from the biological tissues sandwiched between the top portions 92c and 94c along the width direction X.

In consequence, even the biological tissues between the first operation region 82a and the third operation region 84a are prevented from reaching a decomposition temperature in excess of a joining temperature as the biological tissues are away from the top portions 92c and 94c. Therefore, the biological tissues between the first operation region 82a and the third operation region 84a can be adjusted into a joined state (a coagulated state).

As described in the first embodiment, the second and fourth operation regions 82b and 84b can be heated up to a temperature suitable for the joining of the biological tissues L1 and L2 to each other.

The first and third operation regions 82a and 84a are formed into the inclined surfaces 92d and 94d from the top portions 92c and 94c as tops, and hence the decomposition, i.e., cutting (removal) as well as the joining of the biological tissues can be performed by one energy output. At this time, a joining region S of the biological tissues is not limited to the biological tissues between the second operation region 82b and the fourth operation region 84b, but can be enlarged to a part of the biological tissue between the first operation region 82a and the third operation region 84a which is continuous with the biological tissue between the second operation region 82b and the fourth operation region 84b. Therefore, the biological tissues can be joined to each other in a broad range along the width direction X. Needless to say, the biological tissues are joined to each other in a broad range along an axial direction Y. In consequence, according to a treatment system 10 of this embodiment, a strong joining force can be exerted to join the biological tissues to each other.

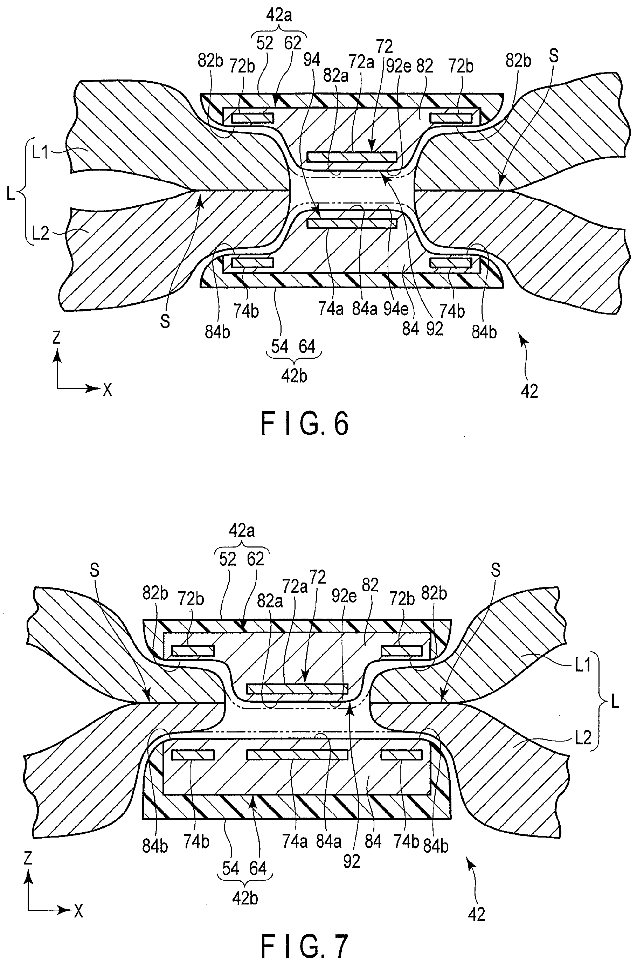

Next, a third embodiment will be described with reference to FIG. 6. This embodiment is a modification of the first and second embodiments, the same members or members having the same functions as those described in the first and second embodiments are denoted with the same reference signs, and detailed description is omitted.

As shown in FIG. 6, a transverse cross section of each of a first operation region 82a of a first energy output portion 62 and a third operation region 84a of a second energy output portion 64 is formed into a substantially trapezoidal shape. That is, the first and third operation regions 82a and 84a have flat surface portions 92e and 94e which face each other. A projecting height of the flat surface portion 92e of the first operation region 82a from the second operation regions 82b and a projecting height of the flat surface portion 94e of the third operation region 84a from fourth operation regions 84b are suitably adjusted, and hence a holding pressure of biological tissues L1 and L2 between the first operation region 82a and the third operation region 84a can be higher than that between the second operation region 82b and the fourth operation region 84b (an outer side from a middle in a width direction X).

It is to be noted that a length of each of the flat surface portions 92e and 94e of the first and third operation regions 82a and 84a which face each other in the width direction X is adjusted, and hence a cut region can easily be adjusted. That is, according to a treatment device 12 of this embodiment, a joined region between the biological tissues in the width direction can be smaller than the state described in the first and second embodiments, but by adjustment of a length of each of the first and third operation regions 82a and 84a in the width direction X (adjustment of a width of each of the flat surface portions 92e and 94e), a length of the cut region in the width direction X and a length of the joined region in the width direction X can suitably be set.

Therefore, each of the first and third operation regions 82a and 84a is formed into a substantially trapezoidal shape having the flat surface portions 92e and 94e, and hence decomposition, i.e., cutting (removal) as well as joining of the biological tissues can be performed by one energy output in the same manner as described in the first and second embodiments.

Next, a fourth embodiment will be described with reference to FIG. 7. This embodiment is a modification of the first to third embodiments, the same members or members having the same functions as those described in the first to third embodiments are denoted with the same reference signs, and detailed description is omitted. This embodiment is especially a modification of the third embodiment.

As shown in FIG. 7, a heat transfer plate 82 of a first energy output portion 62 is formed similarly to the heat transfer plate 82 of the third embodiment. In this embodiment, a third operation region 84a of a heat transfer plate 84 of a second energy output portion 64 is formed into a flat surface flush with fourth operation regions 84b. At this time, a middle of the heat transfer plate 84 of the second energy output portion 64 in a width direction X is substantially controlled at 280°C, and a space between a second operation region 82b and the fourth operation region 84b (an outer side from the middle in the width direction X) is substantially controlled at 200°C. Further, a pressing force in holding a biological tissue can be adjusted in accordance with a projecting amount of a first operation region 82a of the heat transfer plate 82 of the first energy output portion 62 toward the heat transfer plate 84 of the second energy output portion 64.

Therefore, the first operation region 82a is substantially formed into a trapezoidal shape having a flat surface portion 92e, the third operation region 84a is formed into a flat surface flush with the fourth operation regions 84b, and hence decomposition, i.e., cutting (removal) as well as joining of the biological tissues can be performed by one energy output.

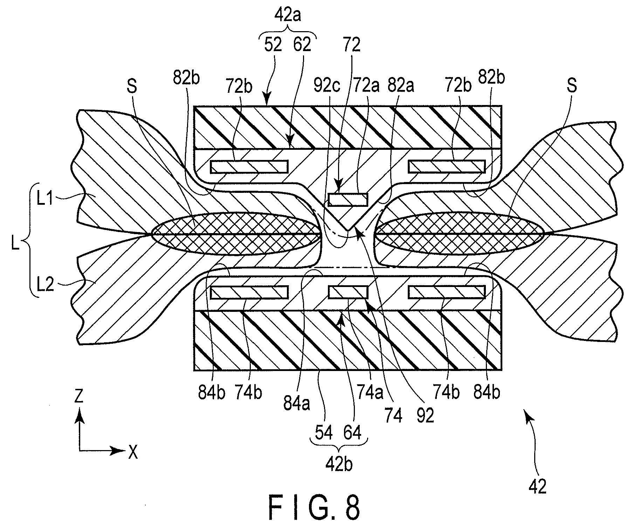

Next, a fifth embodiment will be described with reference to FIG. 8. This embodiment is a modification of the first to fourth embodiments, the same members or members having the same functions as those described in the first to fourth embodiments are denoted with the same reference signs, and detailed description is omitted. This embodiment is especially a modification of the third and fourth embodiments.

As shown in FIG. 8, a transverse cross section of a first operation region 82a of a first energy output portion 62 is substantially triangular. A third operation region 84a of a heat transfer plate 84 of a second energy output portion 64 is formed into a flat surface flush with fourth operation regions 84b in the same manner as in the fourth embodiment.

In consequence, a pressing force in holding a biological tissue can be adjusted in accordance with a projecting amount of the first operation region 82a of a heat transfer plate 82 of the first energy output portion 62 toward the heat transfer plate 84 of the second energy output portion 64.

Here, the transverse cross section of the first operation region 82a is substantially triangularly drawn, but also preferably has the same shape as in the first operation region 82a described in the first embodiment (see FIG. 3B and FIG. 3C) or the same shape as in the first operation region 82a described in the second embodiment (see FIG. 5A and FIG. 5B).

Therefore, the first operation region 82a is substantially formed into a triangular shape having a top portion 92c, the third operation region 84a is formed into a flat surface flush with the fourth operation regions 84b, and decomposition, i.e., cutting (removal) as well as joining of the biological tissues can be performed by one energy output.

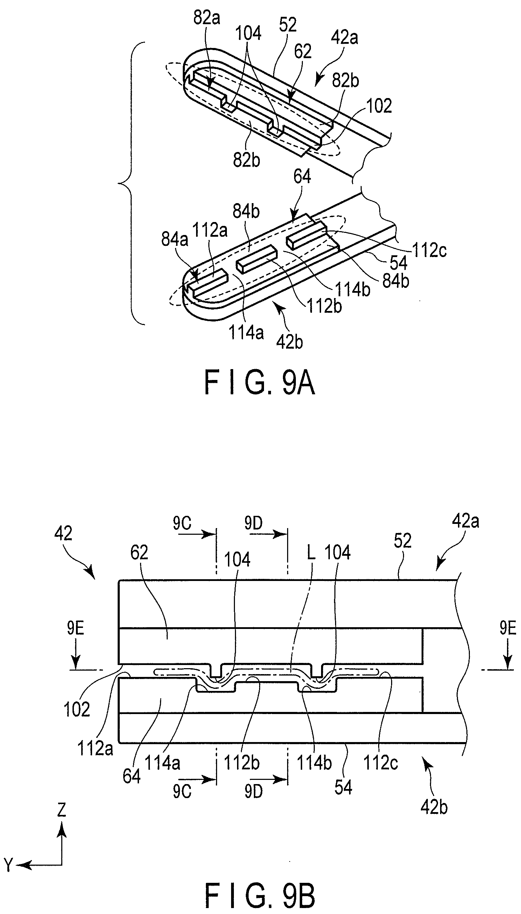

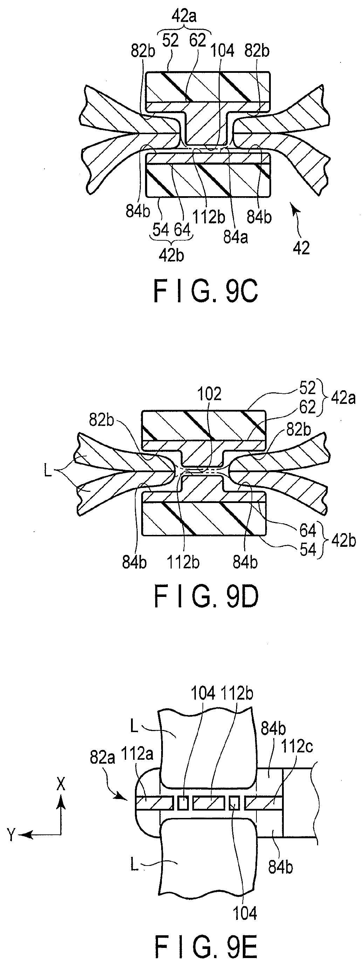

Next, a sixth embodiment will be described with reference to FIG. 9A to FIG. 9E. This embodiment is a modification of the first to fifth embodiments, the same members or members having the same functions as those described in the first to fifth embodiments are denoted with the same reference signs, and detailed description is omitted.

As shown in FIG. 9A to FIG. 9E, a first operation region 82a of a first energy output portion 62 includes a band body 102 extended along a longitudinal axis Y and projected from second operation regions 82b toward a second energy output portion 64 in this embodiment. Further, the first operation region 82a further includes convex portions 104 projected from the band body 102 toward a second energy output portion 64.

As shown in FIG. 9A and FIG. 9C, a third operation region 84a of the second energy output portion 64 has band-like convex portions 112a, 112b and 112c extended along the longitudinal axis Y and projected from fourth operation regions 84b toward the first energy output portion 62. The band-like convex portions 112a, 112b and 112c are coaxially arranged. Between the adjacent band-like convex portions 112a, 112b and 112c, the third operation region 84a is flush with the fourth operation regions 84b. That is, the third operation region 84a has discontinuous portions 114a and 114b. Further, the discontinuous portions 114a and 114b face the convex portions 104 of the first operation region 82a, and the convex portions 104 are disposed in the discontinuous portions. Consequently, as shown in FIG. 9B to FIG. 9E, when a pair of jaws 52 and 54 are closed, the convex portions 104 are received in the discontinuous portions 114a and 114b.

Here, a biological tissue L receives a higher pressure between each convex portion 104 and each of the discontinuous portions 114a and 114b. In consequence, the biological tissue is easy to reach a high temperature and is easily cut. On the other hand, the second operation regions 82b and the fourth operation regions 84b are planar, and therefore hold the biological tissues with a constant pressure to contribute to joining.

Consequently, also in a treatment system 10 according to this embodiment, decomposition, i.e., cutting (removal) as well as the joining of the biological tissues can be performed by one energy output.

Furthermore, the convex portions 104 and the discontinuous portions 114a and 114b apply the pressure to the biological tissue L in a "wedge manner", and hence shift of the biological tissue L during energy treatment can be prevented.

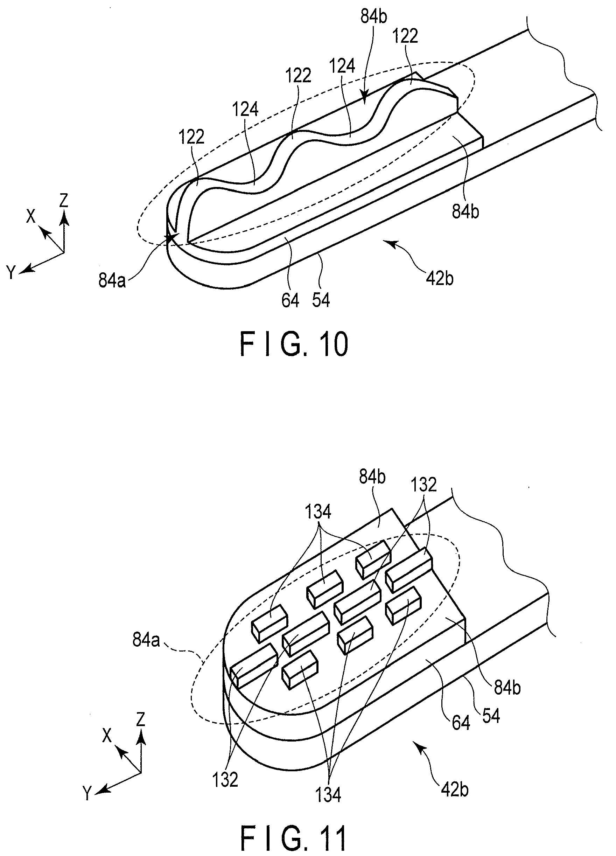

Next, a seventh embodiment will be described with reference to FIG. 10. This embodiment is a modification of the first to sixth embodiments, the same members or members having the same functions as those described in the first to sixth embodiments are denoted with the same reference signs, and detailed description is omitted.

As shown in FIG. 10, a first operation region 82a is formed into a wavy shape in which convex portions 122 and concave portions 124 are smoothly continuous in order along a longitudinal axis. Although not shown in the drawing, in a third operation region 84a, concave portions which face the convex portions 122 and convex portions which face the concave portions 124 are formed along the longitudinal axis. Consequently, in the first and third operation regions 82a and 84a, portions are formed to hold a biological tissue with a pressing force higher than that to hold the biological tissue between a second operation region 82b and a fourth operation region 84b, whereby the biological tissue can easily be cut.

Consequently, also in a treatment system 10 according to this embodiment, decomposition, i.e., the cutting (removal) as well as joining of the biological tissues can be performed by one energy output.

Next, an eighth embodiment will be described with reference to FIG. 11. This embodiment is a modification of the first to seventh embodiments, the same members or members having the same functions as those described in the first to seventh embodiments are denoted with the same reference signs, and detailed description is omitted.

As shown in FIG. 11, a first operation region 82a is formed of three rows along a width direction in this embodiment. In the first operation region 82a, projections (convex portions) 132 are disposed along a longitudinal axis Y. In the first operation region 82a, projections 134 shift from the longitudinal axis Y in a width direction X to be further disposed in parallel with the longitudinal axis Y. That is, convex portions of the first operation region 82a are arranged in rows along the longitudinal axis Y.

In consequence, between a first operation region 82a and a third operation region 84a, portions are formed to hold a biological tissue with a pressing force higher than that to hold the biological tissue between a second operation region 82b and a fourth operation region 84b, whereby the biological tissue can easily be cut.

Consequently, also in a treatment system 10 according to this embodiment, decomposition, i.e., the cutting (removal) as well as joining of the biological tissues can be performed by one energy output.

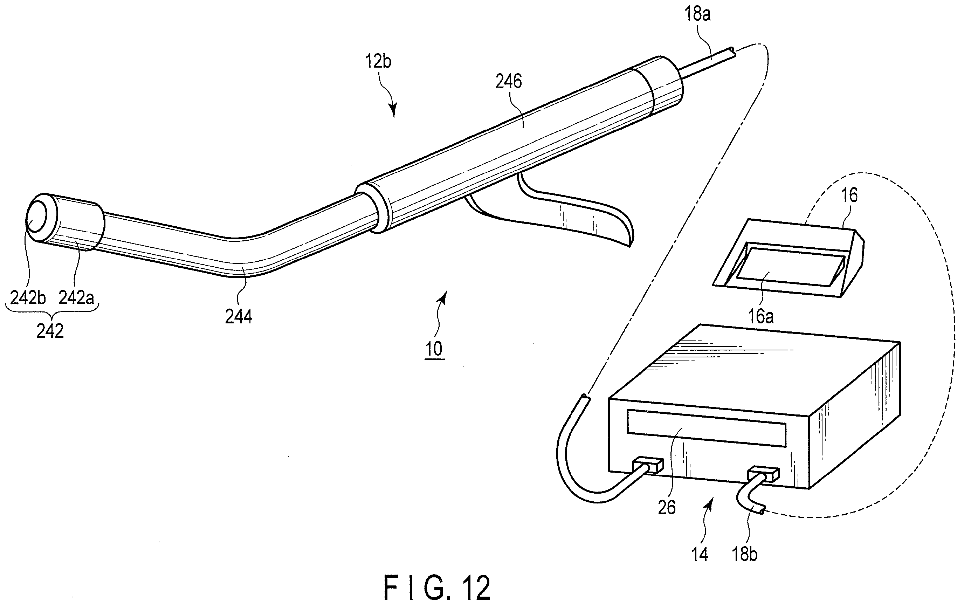

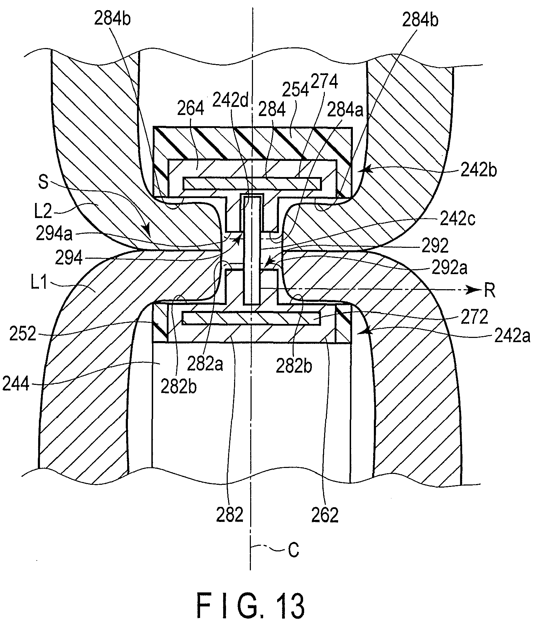

Next, a ninth embodiment will be described with reference to FIG. 12 and FIG. 13. This embodiment is a modification of the first to eighth embodiments, the same members or members having the same functions as those described in the first to eighth embodiments are denoted with the same reference signs, and detailed description is omitted.

As shown in FIG. 12, a treatment device 12b of this embodiment is a circular type. The treatment device 12b includes a treatment section 242 that treats a biological tissue L, a shaft 244, and an operation section 246. The operation section 246 is connected to a controller 14 via a cable 18a.

The shaft 244 is formed into a cylindrical shape. The shaft 244 is appropriately curved in consideration of inserting properties into the biological tissue. Needless to say, the shaft 244 is also preferably linearly formed.

At a distal end of the shaft 244, the treatment section 242 is disposed. The treatment section 242 includes a first treatment section (a main body side treatment section) 242a formed at the distal end of the shaft 244, and a second treatment section (a detachable side treatment section) 242b detachable from the first treatment section 242a.

As shown in FIG. 13, the first treatment section 242a includes a main body side holding portion (a first holding portion) 252 and a first energy output portion 262. The second treatment section 242b includes a detachable side holding portion (a second holding portion) 254, and a second energy output portion 264.

The main body side holding portion 252 and the detachable side holding portion 254 are made of an insulating material. The main body side holding portion 252 is formed into a ring shape in which the first energy output portion 262 is received. The detachable side holding portion 254 is formed into a cap shape in which the second energy output portion 264 is received. The main body side holding portion 252 is integrated with the distal end of the shaft 244, and has a central axis C and a radial direction R perpendicular to the central axis C. The detachable side holding portion 254 has the central axis C and the radial direction R perpendicular to the central axis C.

The first energy output portion 262 includes a first heating portion 272 such as a resistance heating heater, and a first heat transfer plate 282 that transfers heat generated by the first heating portion 272 to the biological tissue. The second energy output portion 264 includes a second heating portion 274 such as the resistance heating heater, and a second heat transfer plate 284 that transfers heat generated by the second heating portion 274 to the biological tissue. The first heat transfer plate 282 of the first treatment section 242a can be coupled with the second heat transfer plate 284 of the second treatment section 242b in a state where the second heat transfer plate 284 of the second treatment section 242b is positioned in a positioning concave portion 242d disposed on the central axis C, by a positioning pin 242c fixed to the first heat transfer plate 282 of the first treatment section 242a on the central axis C. As the positioning pin 242c, there is used a resin material having a heat resistance to withstand a temperature in excess of at least 280°C, or a metal material having an excellent thermal conductivity. When the positioning pin 242c is made of the metal material having the excellent thermal conductivity, heat can be transferred from the first heat transfer plate 282 to the second heat transfer plate 284, and heat can be transferred from the second heat transfer plate 284 to the first heat transfer plate 282.

It is to be noted that, when a pedal 16a of a foot switch 16 is pressed, energy is simultaneously output from an energy output circuit 24 to the first and second energy output portions 262 and 264.

As the first and second heat transfer plates 282 and 284, there is used a metal material such as a stainless steel alloy, or a material having a suitable thermal conductivity, e.g., a fine ceramic material of silicon nitride or the like. As shown in FIG. 13, the first and second heat transfer plates 282 and 284 face each other, and are used as holding surfaces (grasping surfaces) of the biological tissue L as a treatment object. The first heat transfer plate 282 has the central axis C, a first operation region 282a in the vicinity of the central axis, and a second operation region 282b disposed in an outer periphery (on an outer side in the radial direction) of the first operation region 282a. The first and second operation regions 282a and 282b are continuous in the radial direction. The second heat transfer plate 284 has the central axis C, a third operation region 284a in the vicinity of the central axis, and a fourth operation region 284b disposed in an outer periphery (on an outer side in the radial direction) of the third operation region 284a. The third and fourth operation regions 284a and 284b are continuous in the radial direction.

In this embodiment, the first heating portion 272 is planar. Here, the controller 14 raises a temperature of the surface (a grasping surface) of the first operation region 282a of the first heat transfer plate 282 up to about 280°C in, e.g., time t1 (e.g., optional time of several ten seconds to several minutes or the like), and the temperature is controlled to be maintained. Additionally, the controller 14 raises a temperature of the surface of the second operation region 282b of the first heat transfer plate 282 up to about 200°C in, e.g., time t2 (e.g., optional time of several ten seconds to several minutes or the like), and the temperature is controlled to be maintained.

The second heating portion 274 is planar similarly to the first heating portion 272. Here, the controller 14 raises a temperature of the surface (a grasping surface) of the third operation region 284a of the second heat transfer plate 284 up to about 280°C in, e.g., the time t1 (e.g., optional time of several ten seconds to several minutes or the like), and the temperature is controlled to be maintained. Additionally, the controller 14 raises a temperature of the surface of the fourth operation region 284b of the second heat transfer plate 284 up to about 200°C in, e.g., the time t2 (e.g., optional time of several ten seconds to several minutes or the like), and the temperature is controlled to be maintained.

As shown in FIG. 13, in this embodiment, the first operation region 282a includes, on its central axis C, a convex portion 292 disposed closer to the second heat transfer plate 284 than the second operation region 282b. The third operation region 284a includes, on its central axis C, a convex portion 294 disposed closer to the first heat transfer plate 282 than the fourth operation region 284b. In the convex portions 292 and 294, the positioning pin 242c coupling the first and second heat transfer plates 282 and 284 with each other is disposed to couple the plates with each other. It is to be noted that an area of each of top portions 292a and 294a of the convex portions 292 and 294 is sufficiently smaller than that of each of the second and fourth operation regions 282b and 284b.

Next, an operation of a treatment system 10 according to this embodiment will be described.

The main body side treatment section 242a that supports the positioning pin 242c is disposed close to the positioning concave portion 242d of the detachable side treatment section 242b. Further, an end portion (a distal portion from the main body side treatment section 242a) of the positioning pin 242c of the main body side treatment section 242a is fitted into the positioning concave portion 242d to position the detachable side treatment section 242b in the main body side treatment section 242a, thereby coupling the sections. Further, as shown in FIG. 13, biological tissues L1 and L2 as treatment objects are held between the first heat transfer plate 282 and the second heat transfer plate 284.

At this time, a distance between the first operation region 282a of the first heat transfer plate 282 of the first energy output portion 262 and the third operation region 284a of the second heat transfer plate 284 of the second energy output portion 264, i.e., a distance between the convex portions 292 and 294 is smaller than a distance between the second operation region 282b of the first heat transfer plate 282 of the first energy output portion 262 and the fourth operation region 284b of the second heat transfer plate 284 of the second energy output portion 264. Consequently, in the biological tissue, the largest pressing force is applied especially to the biological tissues L1 and L2 between the convex portions 292 and 294 of the first and third operation regions 282a and 284a, and a pressing force between the second operation region 282b and the fourth operation region 284b is smaller than the largest pressing force. Consequently, a holding force to hold the biological tissues L1 and L2 can be larger than that on the outer side in the radial direction, at a position closer to the central axis C of the treatment section 242. Therefore, a close contact force between the biological tissues L1 and L2 is higher than that on the outer side in the radial direction, at the position closer to the central axis C in the radial direction of the treatment section 242.

Here, each of surface temperatures of the convex portion 292 of the first operation region 282a of the first heat transfer plate 282 and the convex portion 294 of the third operation region 284a of the second heat transfer plate 284 is substantially controlled at 280°C. Consequently, outer surfaces of the biological tissues L1 and L2 sandwiched between the first operation region 282a and the third operation region 284a are heated up to 280°C. Further, a boundary of the biological tissues L1 and L2 sandwiched between the first operation region 282a and the third operation region 284a is heated up to about 280°C. Consequently, the biological tissues L1 and L2 sandwiched between the first operation region 282a and the third operation region 284a are decomposed, i.e., burnt out to be incised by the heat transfer plates 282 and 284 whose surface temperatures are 280°C. Therefore, the first and third operation regions 282a and 284a can be adjusted into a state similar to a state where the biological tissues L1 and L2 are cut (removed) circularly based on the central axis C.

The temperature of the outer surface of the biological tissue between the second operation region 282b and the fourth operation region 284b can substantially be heated up to 200°C at a position away from the first and third operation regions 282a and 284a. At this time, the second and fourth operation regions 282b and 284b are formed into flat surfaces facing each other, and hence the outer surface of the biological tissue comes in contact closely with the second and fourth operation regions 282b and 284b, and the boundary between the biological tissues is substantially heated up to about 200°C. In consequence, the second and fourth operation regions 282b and 284b can heat the biological tissues L1 and L2 at a temperature suitable to join the tissues to each other.

Therefore, according to the treatment system 10 of this embodiment, the biological tissue between the first operation region 282a and the third operation region 284a can be decomposed and heated in a state similar to the state where the biological tissue is cut. On the other hand, the biological tissues between the second operation region 282b and the fourth operation region 284b can be heated in a state where the biological tissues are joined to each other. Consequently, according to the treatment system 10 of this embodiment, the decomposition, i.e., the cutting (removal) as well as the joining of the biological tissues can be performed by one energy output. Therefore, the biological tissues can be joined to each other in an annular broad range along the radial direction R on the outer side from the central axis C. Consequently, according to the treatment system 10 of this embodiment, a strong joining force can be exerted to join the biological tissues to each other.

It is to be noted that in this embodiment, there has been described an example where the tubular convex portions 292 and 294 are disposed in the vicinity of the central axis C of the first and second energy output portions 262 and 264, but it is possible to suitably use the stairs described in the first embodiment (see FIG. 3A to FIG. 3C), the gently inclined surfaces described in the second embodiment (see FIG. 5A and FIG. 5B), further the conical shape described in the fifth embodiment (see FIG. 8) or the like. Additionally, the first operation region 282a projecting toward the detachable side treatment section 242b is also preferably formed in the main body side treatment section 242a, and the surface of the heat transfer plate 284 of the detachable side treatment section 242b is also preferably a flat surface. Conversely, the third operation region 284a projecting toward the main body side treatment section 242a is also preferably formed in the detachable side treatment section 242b, and the surface of the heat transfer plate 282 of the main body side treatment section 242a is also preferably a flat surface.

Hitherto, several embodiments have specifically be described with reference to the drawings, but this invention is not limited to the abovementioned embodiments, but includes all implementations to be performed without departing from the gist of the invention.

## Claims

1. A treatment device which applies energy to a biological tissue to treat the biological tissue, the treatment device comprising:
a pair of holding portions that are relatively openable and closable and configured to hold the biological tissue;
a first energy output portion that is disposed in one of the holding portions to face the other holding portion and includes a first operation region that operates to remove the biological tissue as a treatment object when energy is applied, and a second operation region that is disposed around the first operation region and operates to join the biological tissues as the treatment objects when the energy is applied to the second operation region simultaneously with the first operation region; and
a second energy output portion that is disposed in the other holding portion of the holding portions to face the first energy output portion, and that is disposed away from the second operation region more than from the first operation region when the pair of holding portions are relatively closed to hold the biological tissue between the holding portions, wherein a pressing force to be applied to the biological tissue between the second energy output portion and the first operation region is higher than a pressing force to be applied to the biological tissue between the second energy output portion and the second operation region, and the second energy output portion is configured to apply the energy to the biological tissue.

2. The treatment device according to claim 1, wherein a pressing force of the first operation region of the first energy output portion to the biological tissue is larger than a pressing force of the second operation region to the biological tissue in a state where the pair of holding portions are closed to hold the biological tissue by the pair of holding portions.

3. The treatment device according to claim 1, wherein the first operation region projects toward the second energy output portion more than the second operation region.

4. The treatment device according to claim 1, wherein:
the one holding portion has a distal end portion, a proximal end portion, a longitudinal axis defined by the distal end portion and the proximal end portion, and a width direction defined in a direction perpendicular to the longitudinal axis, and
the first operation region includes steps in the width direction.

5. The treatment device according to claim 1, wherein:
the second operation region includes a pair of holding surfaces that hold the biological tissue, and
the first operation region is disposed to be sandwiched between the pair of holding surfaces.

6. The treatment device according to claim 1, wherein:
the second operation region includes a holding surface that holds the biological tissue, and
the first operation region includes convex portions projected from the holding surface toward the second energy output portion.

7. The treatment device according to claim 6, wherein:
the one holding portion has a distal end portion, a proximal end portion and a longitudinal axis defined by the distal end portion and the proximal end portion, and
the convex portions of the first operation region are arranged in rows along the longitudinal axis.

8. The treatment device according to claim 1, wherein the second energy output portion includes a third operation region facing the first operation region of the first energy output portion, and a fourth operation region facing the second operation region of the first energy output portion.

9. The treatment device according to claim 1, wherein the first operation region and the second operation region are continuous.

10. The treatment device according to claim 1, wherein each of the first and second energy output portions includes one heater or heaters.

11. A treatment system comprising:
the treatment device according to claim 1; and
a controller that is connected to the first and second energy output portions of the treatment device and controls temperatures of the first energy output portion and the second energy output portion,
wherein:
each of the first and second operation regions includes a holding surface that holds the biological tissue, and
the controller controls the first operation region and the second energy output portion at a temperature at which the biological tissue between the first operation region and the second energy output portion is removed in the biological tissues, and controls the second operation region and the second energy output portion at a temperature at which the biological tissues between the second operation region and the second energy output portion are joined in the biological tissues, in a state where the biological tissue is held between the first energy output portion and the second energy output portion.

12. The treatment system according to claim 11,
wherein the first energy output portion and the second energy output portion are configured to output heat energy by the controller.

13. The treatment system according to claim 11,
wherein the controller controls a temperature of a surface of the first operation region which comes in contact with the biological tissue at about 280°C, and controls a temperature of a surface of the second operation region which comes in contact with the biological tissue at about 200°C.

14. The treatment system according to claim 11, wherein:
the second energy output portion includes a third operation region facing the first operation region of the first energy output portion, and a fourth operation region facing the second operation region of the first energy output portion, and
the controller controls the first operation region and the third operation region at a temperature at which the biological tissue between the first operation region and the third operation region is removed in the biological tissues, and controls the second operation region and the fourth operation region at a temperature at which the biological tissues between the first operation region and the fourth operation region are joined in the biological tissues, in a state where the biological tissue is held between the first energy output portion and the second energy output portion.

15. The treatment system according to claim 14,
wherein the controller controls temperatures of surfaces of the first and third operation regions which come in contact with the biological tissue at about 280°C, and controls temperatures of surfaces of the second and fourth operation regions which come in contact with the biological tissue at about 200°C.
